# EUROPEAN PATENT APPLICATION

(11) **EP 1 736 800 A1**
(43) Date of publication of application: **27.12.2006**
(21) Application number: 06012371.8
(22) Date of filing: 16.06.2006
(51) Int. Cl.: G01S 15/89

(54) **Ultrasound diagnostic system with an external mass storage unit**

(30) Priority: 16.06.2005 KR 20050051676
(71) Applicant: MEDISON CO., LTD., Kangwon-do 250-870 (KR)
(72) Inventor: Hyun, Dong Gyu, Gangnam-gu Seoul 135-280 (KR)
(74) Representative: Lorenz, Werner

(57) **Abstract**

The present invention is directed to an ultrasound diagnostic system, including: an ultrasound transmission/reception unit for transmitting ultrasound signals to a target object and receiving ultrasound echo signals reflected from the target object; a data forming unit for forming ultrasound data based on the ultrasound echo signals, the data forming unit including a first interface unit for outputting the ultrasound data; and an external storage unit for storing the ultrasound data outputted from the data forming unit, the external storage unit including a second interface unit for receiving the ultrasound data.

## Description

The present invention generally relates to an ultrasound diagnostic system, and more particularly to an ultrasound diagnostic system having an external mass storage unit for storing a large quantity of ultrasound data such as live 3-dimensional ultrasound data.

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., organs of a human patient). The ultrasound diagnostic system generally uses a wide bandwidth transducer to transmit and receive ultrasound signals. The ultrasound diagnostic system forms images of the internal tissues of a human body by electrically exciting an acoustic transducer element or an array of acoustic transducer elements to generate ultrasound pulses that travel into the body. The ultrasound pulses produce ultrasound echoes since they reflect from body tissues, which appear as discontinuities to the propagating ultrasound pulses. The various ultrasound echoes return to the transducer and are converted into electrical signals, which are amplified and processed to produce ultrasound data for an image of the tissues. This ultrasound diagnostic system is of significant importance to the medical field since it provides physicians with real-time high-resolution images of internal features of a human anatomy without the need for invasive observation techniques such as surgery.

The ultrasound data, which are obtained based on the ultrasound echo signals, should be temporarily stored in order to provide an ultrasound image in the ultrasound diagnostic system. Conventionally, the ultrasound data are stored in a memory built in the ultrasound diagnostic system. The ultrasound data include analog or digital ultrasound image data and image parameters related to the ultrasound image data. However, since the capacity of the memory used in the conventional ultrasound diagnostic system is small, the use of such memory is quite limited when dealing with a significantly large quantity of ultrasound data such as live 3-dimensional ultrasound data.

Additional memories may be installed in the ultrasound diagnostic system in order to overcome the capacity limitation of the memory. Obviously, this is to enhance the overall memory capacity of the ultrasound diagnostic system. However, there are problems in that the number of memories, which can be additionally built into the ultrasound diagnostic system, is restricted since the size of the ultrasound diagnostic system is limited and the memory is quite expensive.

In order to overcome the limitations associated with storage capacity and memory enhancement as described above, the ultrasound data are stored in a magnetic record medium, which is connected to the ultrasound diagnostic system, in an analog format. Alternatively, such data can be stored in a compact disk in a digital format, which is applied to a compact disk player connected to the ultrasound diagnostic system. However, when the ultrasound data are stored in the magnetic record medium in the analog format, it is difficult to display high-quality ultrasound images and search for desired ultrasound data in a rapid manner. Also, it is further difficult to permanently store the ultrasound data.

In case of storing the ultrasound data in the compact disk, finalization and un-finalization aligning tracks and sectors of the compact disk should be carried out whenever the ultrasound information are recorded in the compact disk. Thus, a considerable amount of time is typically consumed to record the ultrasound data. Therefore, there are problems in that it is impossible to record the ultrasound data in real time during an examination. Further, it becomes very inconvenient to do so because the ultrasound data must be stored in an auxiliary storing medium. The ultrasound data stored in the auxiliary storing medium must then be edited such that the ultrasound data can be recorded in the compact disk.

The present invention provides an ultrasound diagnostic system, which has a mass storage unit capable of storing a large quantity of ultrasound data in real time and easily searching desirable ultrasound data.

According to one aspect of the present invention, there is provided an ultrasound diagnostic system, including: an ultrasound transmission/reception unit for transmitting ultrasound signals to a target object and receiving ultrasound echo signals reflected from the target object; a data forming unit for forming ultrasound data based on the ultrasound echo signals, the data forming unit including a first interface unit for outputting the ultrasound data; and an external storage unit for storing the ultrasound data outputted from the data forming unit, the external storage unit including a second interface unit for receiving the ultrasound data.

According to another aspect of the present invention, there is provided a method of storing ultrasound data in an external storing unit in an ultrasound diagnostic system, including the steps of: a) forming ultrasound data based on ultrasound echo signals reflected from a target object, the ultrasound data including ultrasound image data and image parameters related to the ultrasound image data; b) selecting a data storing mode for storing the ultrasound data; c) assigning identification to the ultrasound image data; d) transmitting the ultrasound data to an external storing unit; e) checking whether to compress the ultrasound image data; f) compressing the ultrasound image data; and g) storing the ultrasound image data together with the image parameters.

According to still another aspect of the present invention, there is provided a method of reading out ultrasound data from an external ultrasound unit in an ultrasound diagnostic system, including the steps of: a) selecting a data reading mode; b) searching desirable ultrasound data in an external storing unit, the ultrasound data including ultrasound image data and image parameters related to the ultrasound image data; c) checking whether to decompress the ultrasound image data; d) decompressing the ultrasound image data; and e) outputting the ultrasound image data together with the image parameters.

The above and other objects and features of the present invention will become apparent from the following description of preferred embodiments given in conjunction with the accompanying drawings, in which:
Fig. 1 is a block diagram illustrating an ultrasound diagnostic system constructed in accordance with a preferred embodiment of the present invention;
Fig. 2 is a block diagram illustrating an external storing unit constructed in accordance with a preferred embodiment of the present invention;
Fig. 3 is a flow chart showing a process of storing ultrasound data in an external storing unit constructed in accordance with a preferred embodiment of the present invention; and
Fig. 4 is a flow chart showing a process of reading out ultrasound data stored in an external storing unit constructed in accordance with a preferred embodiment of the present invention.

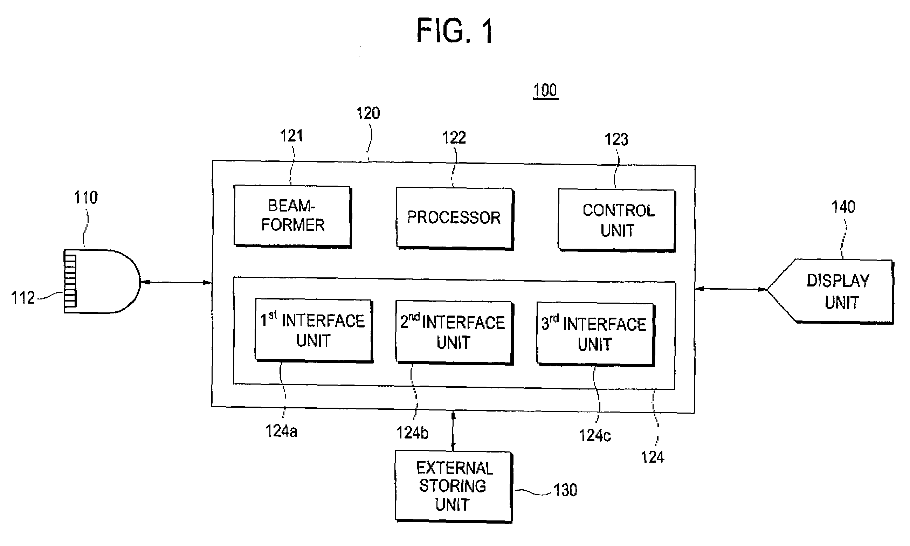

Fig. 1 is a block diagram showing an ultrasound diagnostic system 100, which is constructed in accordance with a first embodiment of the present invention. As shown in Fig. 1, the ultrasound diagnostic system 100 includes a probe 110, a body 120, an external storing unit 130, a display unit 140 and an input unit 150. The probe 110 includes a 1-dimensional or 2-dimensional array transducer 112. The transmit signals, which are appropriately delayed so as to form a focused ultrasound beam in a beamformer 121 included in the body 120, are transmitted to the array transducer 112. The focused ultrasound beam is then transmitted along a scan line of a target object (not shown). The probe 110 receives ultrasound echo signals received from the target object and converts the ultrasound echo signals into electrical signals (hereinafter referred to as receive signals). The receive signals are then transferred to the beamformer 121.

The body 120 further includes an image processor 122, a control unit 123 and an interface unit 124. The beamformer 121 controls the delays of transmit signals, which arc to be transmitted to the array transducer 112. This is so that the ultrasound signals outputted from the array transducer 112 can be focused on a focal point. Also, the beamformer 121 focuses the receive signals transmitted from the array transducer 112 in consideration of the delays, which the echo signals are arrived at the respective transducers in the beamformer 121, thereby producing ultrasound raw data

The image processor 122 converts the raw image ultrasound data into digital data for digital signal processing and performs various kinds of digital signal processing for the digital data, thereby producing digital ultrasound data. The digital ultrasound data contains digital ultrasound image data and image parameters related to the digital ultrasound image data. The digital ultrasound image data may include 2-dimensional data, Doppler image data, Doppler spectrum data, M-mode image data, B-mode image data, ECG (electrocardiogram) data and the like. The image parameters include data synchronization information necessary for synchronizing the ultrasound image data, which are different kinds of data such as the 2-dimensional image data and the Doppler spectrum data, acquisition time interval information between frames in the ultrasound image data, image processing information necessary for processing the ultrasound image data and data indication information for indicating data information of the ultrasound image data. The data processing information may include a gain, a post curve, a scale, a base line, a display mode, a display size and frame average of the ultrasound image data. The data indication information may include the information related to a color box, a depth meter, a sample volume line and an M line in the ultrasound image data. Further, the image processor 122 performs post-processing for the ultrasound image data read out from the external storing unit 130.

The control unit 123 controls the entire operations of the ultrasound diagnostic system 100. For example, the control unit 123 ensures that the ultrasound data outputted from the image processor 122 are displayed on a display unit 140 or stored in the external storing unit 130 depending on the operator's choice. Also, the control unit 123 ensures that the ultrasound data stored in the external storing unit 130 are read out. Further, the control unit 123 assigns identifications (IDs) to the respective ultrasound data to be stored in the external storing unit 130. The IDs are assigned differently according to the type of data.

The ultrasound data may be outputted through the interface unit 124 in a fixed period such as via NTSC, PAL, progressive scan or the like. Also, the ultrasound data may be outputted in a specific period depending on the type of ultrasound data. The interface unit 124 includes a first interface unit 124a, a second interface unit 124b and a third interface unit 124c. The first interface unit 124a receives the ultrasound image data and transfers the ultrasound image data to the external storing unit 130. The ultrasound image data include the ultrasound raw data outputted from the beamformer 121 and the digital ultrasound image data outputted from the image processor 122. The first interface unit 124a may be a terminal selected from the group consisting of a VHS terminal, an S-VHS terminal and a D-sub terminal. The second interface unit 124b receives the image parameters, which are outputted from the image processor 122, related to the digital ultrasound image data and transfers the image parameters to the external storing unit 130. The second interface unit 124b may be one of Ethernet, RS-232C, USB, IEEE1394, Bluetooth, infrared port and the like. The third interface unit 124c is for transmitting the control signals to the external storing unit 130 to control the operation of the external storing unit 130. The third interface unit 124c may be one of Ethernet, RS-232C, USB, IFEE1394, blue-tooth, infrared port and the like (similar to the second interface unit 124b).

The external storing unit 130 receives the ultrasound data from the interface unit 124 in a fixed period such as via NTSC, PAL, progressive scan or the like. Also, the external storing unit 130 receives the ultrasound data from the interface unit 124 in a specific period depending on the type of ultrasound data. The external storing unit 130 stores the ultrasound image data including the ultrasound raw data and digital ultrasound image data and the image parameters. The external storing unit 130 may be selected from the group consisting of a hard disk dive (HDD), a digital versatile disk (DVD) and a flash memory.

The display unit 140 receives the digital ultrasound data from the image processor 122 and displays an ultrasound image based on the digital ultrasound data.

The input unit 150 receives the inputs of an operator. The control unit 123 controls the operations of the ultrasound diagnostic system 100 in response to the operator's inputs. That is, the control unit 123 generates a plurality of control signals in response to the inputs of the operator for controlling each element constructing the ultrasound diagnostic system 100.

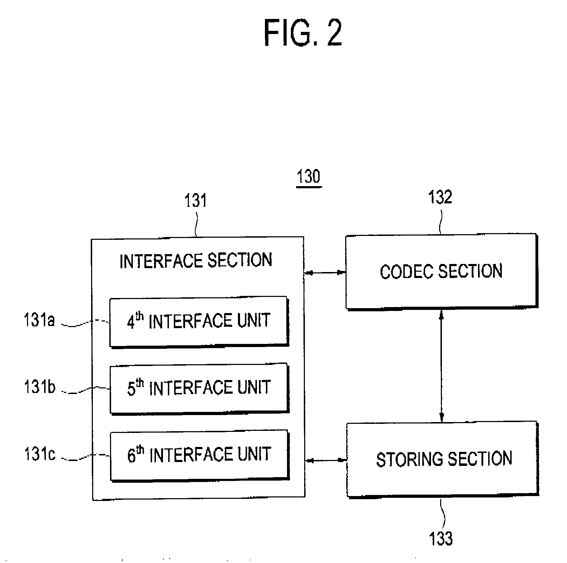

Hereinafter, the structure and operation of the external storing unit 130 will be described below with reference to Fig. 2.

Fig. 2 is a block diagram illustrating the external storing unit, which is constructed in accordance with the preferred embodiment of the present invention. As shown in Fig. 2, the external storing unit 130 includes an interface section 131, a codec section 132 and a storing section 133. The interface section 131 includes a fourth interface unit 131a, a fifth interface unit 131b and a sixth interface unit 132c. The fourth interface unit 131a, which is connected to the first interface unit 124a included in the body 120, receives the ultrasound image data and transfers the ultrasound image data to the storing section 133. The ultrasound image data are stored together with the IDs assigned by the control unit 123 in the storing section 133. The ultrasound image data may be compressed in the codec section 132 for reducing the amount of data before being stored in the storing section 133 under the control signal outputted from the control unit 123. When the ultrasound image data are compressed in the codec section 132, compression information is also stored in the storing section 133 together with the compressed ultrasound image data. The fourth interface unit 131a may be selected from the group consisting of a VHS terminal, an S-VHS terminal and a D-sub terminal.

The fifth interface unit 131b, which is connected to the second interface unit 124b, receives the image parameters related to the ultrasound image data and transfers the image parameters to the storing section 133. The sixth interface unit 131c, which is connected to the third interface unit 124c, receives the control signals. The fifth interface unit 131b and the sixth interface unit 131c may be selected from the group consisting of Ethernet, RS-232C, USB, IEEE1394, blue-tooth, infrared port and the like.

The codec section 132 is used to compress the ultrasound image data, which are to be stored in the storing section 133, and decompresses the compressed ultrasound image data read out from the storing section 133.

The storing section 133 stores the ultrasound data including the ultrasound image data transferred through the fourth interface unit 131a and the image parameters transferred through the fifth interface unit 131b in response to the control signal received at the sixth interface unit 131c.

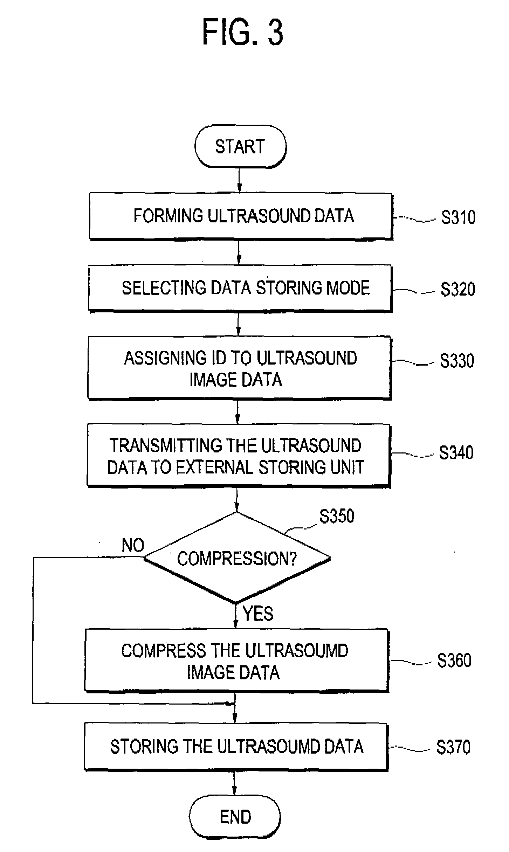

The procedure for storing the ultrasound data in the external storing unit 130 will be described below in view of Fig. 3.

Fig. 3 is a flow chart showing the process of storing the ultrasound data in the external storing unit 130, which is constructed in accordance with the preferred embodiment of the present invention. As shown in Fig. 3, the ultrasound data including the ultrasound raw data, the digital ultrasound image data and the image parameters are formed at step S310. If the operator selects a data storing mode for storing the ultrasound data in the external storing unit 130 at step S320, then the control unit 123 assigns ID to the ultrasound image data at step S330.

Thereafter, the ultrasound image data are transmitted to the fourth interface unit 131a of the external storing unit 130 through the first interface unit 124a and the image parameters are transmitted to the fifth interface unit 131b of the external storing unit 130 through the second interface unit 124b at step S340. It is then checked whether the ultrasound image data are to be compressed according to the control signal transmitted to the sixth interface unit 131c at step S350. If it is determined that the ultrasound image data should be compressed, then the ultrasound image data are compressed in the codec section 132 at step S360. The compressed ultrasound image data are stored in the storing section 133 together with the image parameter at step S370.

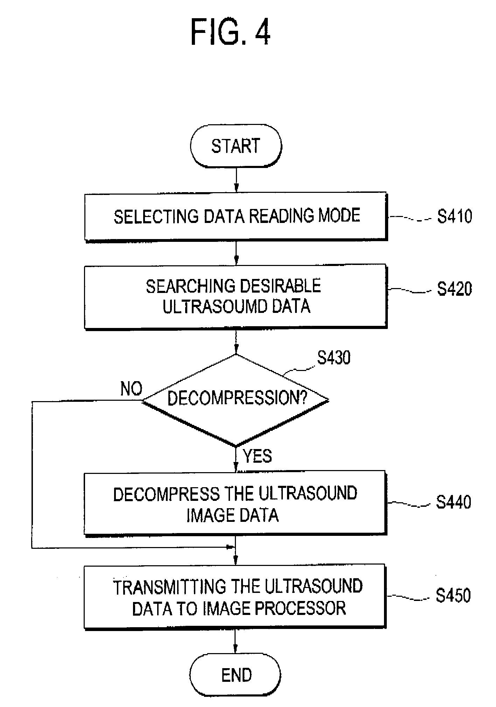

The procedure for reading out the ultrasound data stored in the external storing unit 130 will be described below in view of Fig. 4.

Fig. 4 is a flow chart showing a process of reading out the ultrasound data stored in the external storing unit 130, which is constructed in accordance with the preferred embodiment of the present invention. As shown in Fig. 4, if the operator selects a data read mode for reading out the ultrasound data from the storing unit 130 through the input unit 150 at step S410, then the control unit 123 transmits a control signal to the external storing unit 130 through the third interface unit 124c and the sixth interface unit 131 c for searching the ultrasound image data and the image parameters, which correspond to the selection of the operator at step S420. The searching process is first carried out for the image parameters. After searching the image parameter corresponding to the selection of the operator, the searching process is carried out for the ultrasound data by identifying ID of the ultrasound image data, which corresponds to the searched image parameter. A portion of the ultrasound image data may be searched according to the selection of the operator. For example, specific ECG data from the third ECG peak to the sixth ECG peak may be searched in the ECG data stored in the storing section 133 according to the selection of the operator. Subsequently, it is checked whether the ultrasound image data should be decompressed at step S430. If the ultrasound image data are the compressed data, then the ultrasound image data are decompressed in the codec section 132 at step S440. The searched ultrasound image data are transmitted to the image processor 122 through the fourth interface unit 131 a and the first interface unit 124a at step S450. Further, the searched image parameters are transmitted to the image processor 122 through the fifth interface unit 131b and the second interface unit 124b at step S460.

In accordance with the preferred embodiment of the present invention, the ultrasound data may be stored in the external storing unit 130 during a specific time or as a specific amount of data according to the input of the operator. Also, the ultrasound data may be stored in the storing unit 130 according to a specific event. For example, the ECG data corresponding to 3 times of ECG trigger signals may be stored in the external storing unit 130 according to the input of the operator. Further, the recently acquired ultrasound data may be stored for a predetermined time without the operator's request in accordance with another embodiment of the present invention.

Further, since the ultrasound data are stored with ID of the ultrasound image data and the image parameters indicating information related to the ultrasound image data, the operator can easily search the desirable ultrasound data in the external storing unit 130.

As described above, since the external storing unit capable of storing an enormous quantity of ultrasound data is adopted in the ultrasound diagnostic system of the present invention, the ultrasound data formed based on the ultrasound echo signals can be stored in real time and easily searched according to the request of the operator.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hercto.

## Claims

1. An ultrasound diagnostic system, comprising:
an ultrasound transmission/reception unit for transmitting ultrasound signals to a target object and receiving ultrasound echo signals reflected from the target object;
a data forming unit for forming ultrasound data based on the ultrasound echo signals, the data forming unit including a first interface unit for outputting the ultrasound data; and
an external storage unit for storing the ultrasound data outputted from the data forming unit, the external storage unit including a second interface unit for receiving the ultrasound data.

2. The ultrasound diagnostic system of Claim 1, wherein the ultrasound data includes ultrasound image data and image parameters related to the ultrasound image data.

3. The ultrasound diagnostic system of Claim 1, wherein the ultrasound image data include 2-dimensional data, Doppler image data, Doppler spectrum data, M-mode image data, B-mode image data and ECG (electrocardiogram) data, and wherein the image parameters include synchronization information necessary for synchronizing ultrasound image data, time interval information acquiring each frame in the ultrasound image data, image processing information necessary for processing the ultrasound image data and data indication information for indicating data information of the ultrasound image data.

4. The ultrasound diagnostic system of Claim 2, further comprising a control unit for generating a control signal according to an input of an operator and assigning identification to the ultrasound image data.

5. The ultrasound diagnostic system of Claim 4, wherein the first interface unit includes:
a first interface section for receiving and transmitting the ultrasound image data;
a second interface section for receiving and transmitting the image parameters; and
a third interface section for transmitting the control signal;
wherein the second interface unit includes:
a fourth interface section for receiving and transmitting the ultrasound image data;
a fifth interface section for receiving and transmitting the image parameters; and
a sixth interface section for receiving the control signal.

6. The ultrasound diagnostic system of Claim 5, wherein the external storing unit further includes:
a codec section for compressing and decompressing the ultrasound image data in response to the control signal; and
a storing section for storing the ultrasound image data, the compressed ultrasound image data and the image parameter.

7. A method of storing ultrasound data in an external storing unit in an ultrasound diagnostic system, comprising the steps of:
a) forming ultrasound data based on ultrasound echo signals reflected from a target object, the ultrasound data including ultrasound image data and image parameters related to the ultrasound image data;
b) selecting a data storing mode for storing the ultrasound data;
c) assigning identification to the ultrasound image data;
d) transmitting the ultrasound data to an external storing unit;
e) checking whether to compress the ultrasound image data;
f) compressing the ultrasound image data; and
g) storing the ultrasound image data together with the image parameters.

8. The method of Claim 7, wherein the ultrasound image data include 2-dimensional data, Doppler image data, Doppler spectrum data, M-mode image data, B-modc image data and ECG data, and wherein the image parameters include synchronization information necessary for synchronizing ultrasound image data, time interval information acquiring each frame in the ultrasound image data, image processing information necessary for processing the ultrasound image data and data indication information for indicating data information of the ultrasound image data.

9. A method of reading out ultrasound data from an external ultrasound unit in an ultrasound diagnostic system, comprising the steps of:
a) selecting a data reading mode;
b) searching desirable ultrasound data in an external storing unit, the ultrasound data including ultrasound image data and image parameters related to the ultrasound image data;
c) checking whether to decompress the ultrasound image data;
d) decompressing the ultrasound image data; and
e) outputting the ultrasound image data together with the image parameters.

10. The method of Claim 9, wherein the ultrasound image data include 2-dimensional data, Doppler image data, Doppler spectrum data, M-mode image data, B-mode image data and ECG data, and wherein the image parameters include synchronization information necessary for synchronizing ultrasound image data, time interval information acquiring each frame in the ultrasound image data, image processing information necessary for processing the ultrasound image data and data indication information for indicating data information of the ultrasound image data.
